# EUROPEAN PATENT APPLICATION

(11) **EP 4 755 405 A2**
(43) Date of publication of application: **10.06.2026**
(21) Application number: 25219932.8
(22) Date of filing: 02.12.2025
(51) Int. Cl.: A61L 2/26, B25J 21/02, D06F 1/00, E06B 7/23

(54) **MACHINE FOR WASHING AND/OR DECONTAMINATING OBJECTS, FOR APPLICATIONS IN THE CHEMICAL, PHARMACEUTICAL AND/OR CLINICAL/PRECLINICAL RESEARCH FIELDS, AND METHOD OF OPERATION OF SAID MACHINE**

(30) Priority: 03.12.2024 IT 202400027273
(71) Applicant: IWT Srl, 21020 Casale Litta (Varese) (IT)
(72) Inventor: IAMETTI, Christian, 21020 Vinago di Mornago (VA) (IT); PERARI, Andrea, 28041 Arona (NO) (IT)
(74) Representative: Metroconsult Srl

(57) **Abstract**

Machine for washing and/or decontaminating objects, for applications in the chemical, pharmaceutical and/or clinical/preclinical research fields, comprising: a box-shaped body (10), defining a treatment chamber (20), said treatment chamber (20) having at least one entrance (30); a door (40), associated with said entrance (30) and controllable between a closed condition, in which it closes said entrance (30), and an open condition, in which it does not close said entrance (30); an inflatable gasket (50), extending along a perimeter of said door (40), said inflatable gasket (50) being controllable between an inflated configuration, in which it generates a sealed closure when said door (40) is in the closed condition, and a deflated configuration; at least one latch (60), controllable between an operating condition, in which it locks the door (40) in the closed condition, and a rest condition, in which it exerts no locking action; a pneumatic circuit (100), acting upon said inflatable gasket (50) to bring it into the inflated configuration. Said at least one latch (60) houses part of said pneumatic circuit (100).

## Description

### Technical field

The present invention relates to a machine for washing, disinfecting, sterilizing, decontaminating, containing and/or isolating products, particularly for use in the pharmaceutical industry.

The invention further relates to a method of operation of said machine.

### Background art

Some machines typically used in the clinical/preclinical research, chemical and pharmaceutical fields are provided with at least one entrance/exit door for a load to be subjected to specific treatments. For example, such treatments may include washing, disinfection/sterilization, containment, isolation, etc.

Applications of such machines include, for example, treatments of laboratory breeding cages for clinical/preclinical research, or processes executed in the chemical and pharmaceutical industries.

The machines employed for such processes are essentially containment chambers into which a load is placed, which is to be processed. As mentioned above, such chambers are equipped with at least one door for loading and unloading the material to be processed. The door typically has one or more hinges allowing it to be opened and closed.

On at least one side of the door, typically the one opposite the hinged side, there is typically at least one latch for locking the door.

The door perimeter is fitted with an inflatable hollow tubular gasket, which is pressurized when the door is in the closed position, thus ensuring a hermetic seal against a seat perimeter coinciding with the fixed frame of the chamber, i.e. of the machine, that surrounds the door.

In order that the gasket can be inflated to ensure a hermetic seal of the door when the machine is in operation, at least one of the hinges is provided with an internal passage for fluid under pressure: when the door is closed and locked by the latch, a pneumatic system lets air flow under pressure into the gasket through said hinge, thereby inflating the gasket up to a predetermined pressure required to guarantee an air-tight seal.

The Applicant observes that, while it is functionally satisfactory, the known solution summarized above suffers from a few undesired drawbacks and effects.

In particular:
- the hinge that incorporates the air passage is complex and costly to manufacture;
- the seals internal to the hinge are difficult to check and maintain, in addition to being subject to mechanical stress and damage during the rotation;
- the pressurization of the perimetric tubular gasket must be synchronized with the actual closure of the latch, and this requires a non-negligible level of complexity in the control logic of the machine, since suitable pressure switches have to be employed to detect such synchronization and to inform the system that is monitoring the process;
- in case of emergency (i.e. in the event that the door needs to be opened during the process), it is not easy to interrupt said synchronization and restore it after the problem has been solved; in particular, the necessity of two separate actions (one for deflating the gasket, and one for opening the latch) results in more complex detection and control of the safety circuit: as a matter of fact, a sensor is needed to detect the closed door condition, another sensor is needed to detect the closed latch condition, and yet another sensor is needed to detect the inflated gasket condition;
- the circuit through the hinge is a throttled labyrinth that generates considerable load losses, resulting in longer gasket pressurization and, most importantly, depressurization times;
- if, when the door is open, the gasket is inadvertently inflated, the latter may, since it finds no resistance from the frame, get damaged or even explode;
- for chamber configurations allowing access to an operator, for safety reasons it is necessary that the door can be opened from the inside in case of emergency to allow the operator to escape, also in the event of pneumatic failure or electric outage, and the currently known configuration makes it difficult and expensive to meet this need.

Therefore, the Applicant intends to provide the machines used in the above-mentioned fields with a technology that can overcome, or at least limit, such drawbacks.

### Summary of the invention

In accordance with a first aspect, the invention relates to a machine for washing and/or decontaminating objects, for applications in the chemical, pharmaceutical and/or clinical/preclinical research fields.

Preferably, said machine comprises a box-shaped body.

Preferably, said box-shaped body defines a treatment chamber.

Preferably, said treatment chamber has at least one entrance.

Preferably, said machine comprises a door.

Preferably, said door is associated with said entrance.

Preferably, said door is controllable into a closed condition.

Preferably, when said door is in the closed condition, it closes said entrance.

Preferably, said door is controllable into an open condition.

Preferably, when said door is in the open condition, it does not close said entrance.

Preferably, said machine comprises an inflatable gasket.

Preferably, said inflatable gasket extends along a perimeter of said door.

Preferably, said inflatable gasket is controllable into an inflated configuration.

Preferably, when said inflatable gasket is in the inflated configuration, it generates a sealed closure when said door is in the closed condition.

Preferably, said inflatable gasket is controllable into a deflated configuration.

Preferably, said machine comprises at least one latch.

Preferably, said at least one latch is controllable into an operating condition.

Preferably, when said at least one latch is in the operating condition, it locks the door in the closed condition.

Preferably, said at least one latch is controllable into a rest condition.

Preferably, when said at least one latch is in the rest condition, it exerts no locking action.

Preferably, said machine comprises a pneumatic circuit.

Preferably, said pneumatic circuit acts upon said inflatable gasket.

Preferably, said pneumatic circuit brings said inflatable gasket into the inflated configuration.

Preferably, said at least one latch houses part of said pneumatic circuit.

In accordance with a second aspect, the invention relates to a method of operation of a machine according to the first aspect.

Preferably, said method comprises bringing said door into said closed condition.

Preferably, said method comprises bringing said at least one latch into the operating condition, so as to lock said door in the closed condition.

Preferably, said method comprises activating said pneumatic circuit.

Preferably, said pneumatic circuit is activated to bring said inflatable gasket into the inflated configuration.

Preferably, said inflatable gasket is brought into the inflated configuration through said at least one latch.

Preferably, when said inflatable gasket is in the inflated configuration, it generates a sealed closure of said door.

In one or more of the above aspects, the invention may comprise one or more of the following preferred features.

Preferably, said at least one latch, when it is in the operating condition, allows for fluidic communication between a source of fluid under pressure and said inflatable gasket in order to bring the latter into the inflated configuration.

Preferably, said at least one latch, when it is in the rest condition, does not allow for fluidic communication between the source of fluid under pressure and the inflatable gasket.

Preferably, said at least one latch has an inner cavity.

Preferably, said inner cavity has a first aperture.

Preferably, said inner cavity has a second aperture.

Preferably, said first aperture is connected to said source of fluid under pressure.

Preferably, said second aperture is in fluidic communication with said inflatable gasket when the at least one latch is in the operating condition.

Preferably, said pneumatic circuit comprises a valve assembly.

Preferably, said valve assembly is configured to selectively allow fluid under pressure to flow from the source to the inflatable gasket.

Preferably, said machine comprises an actuator.

Preferably, said actuator is configured to move said at least one latch between the operating condition and the rest condition.

Preferably, said actuator is a linear actuator.

Preferably, said actuator is equipped with a drive unit.

Preferably, said actuator is equipped with a sliding element.

Preferably, said sliding element is driven by said drive unit.

Preferably, said at least one latch is connected to said sliding element.

Preferably, said at least one latch is connected to said sliding element through an elongate element.

Preferably, said elongate element has a substantially central portion.

Preferably, said elongate element has a first end.

Preferably, said elongate element has a second end.

Preferably, said second end is opposite said first end.

Preferably, the sliding element is engaged with the substantially central portion of the elongate element.

Preferably, the at least one latch is engaged with the first end of the elongate element.

Preferably, said machine comprises an alignment element.

Preferably, said alignment element is coupled to the second end of the elongate element.

Preferably, said actuator is a pneumatic actuator.

Preferably, said valve assembly is configured to be driven into a first condition.

Preferably, when said valve assembly is in the first condition, it directs a flow of fluid towards said actuator to bring said at least one latch into the rest condition.

Preferably, said valve assembly is configured to be driven into a second condition.

Preferably, when said valve assembly is in the second condition, it directs a flow of fluid towards said actuator to bring said at least one latch into the operating condition.

Preferably, said valve assembly is configured to be driven into a third condition.

Preferably, when said valve assembly is in the third condition, it directs a flow of fluid towards said at least one latch to bring said inflatable gasket into the inflated configuration.

Preferably, said at least one latch is mounted to said box-shaped body.

Preferably, when said at least one latch is in said operating condition, it intercepts a portion of said door so as to exert said locking action.

Preferably, said at least one latch has a substantially cylindrical shape.

Preferably, said at least one latch has a tapered longitudinal end.

Preferably, said tapered longitudinal end facilitates the movement into the operating condition.

Preferably, said machine comprises an auxiliary gasket.

Preferably, said auxiliary gasket is associated with said at least one latch.

Preferably, said auxiliary gasket provides a seal between the pneumatic circuit and the inflatable gasket when the latch is in the operating condition.

Preferably, said machine comprises a striker block.

Preferably, said striker block is in fluidic communication with said inflatable gasket.

Preferably, when the at least one latch is in the operating condition, said at least one latch is in abutment with said striker block.

Preferably, when the at least one latch is in the operating condition, said striker block allows for fluidic communication between the at least one latch and the inflatable gasket.

Preferably, said striker block is at least partly shaped to match said at least one latch.

Preferably, said auxiliary gasket is mounted to said at least one latch.

Preferably, said auxiliary gasket is mounted to said striker block.

Preferably, said auxiliary gasket provides a seal between said at least one latch and said striker block.

Preferably, when said at least one latch is in the rest condition, it is spaced apart from said striker block.

Preferably, when said at least one latch is in the rest condition, it is not in abutment with said striker block.

Preferably, said inflatable gasket is housed in the perimeter of the door.

Preferably, said inflatable gasket is housed in a perimetric groove of said door.

Preferably, said method comprises a step of opening said door.

Preferably, said step of opening comprises moving said at least one latch from the operating condition to the rest condition.

Preferably, said step of opening comprises bringing said inflatable gasket into the deflated configuration.

Preferably, said step of opening comprises moving said door from the closed condition to the open condition.

Preferably, said method comprises monitoring a pressure within said inflatable gasket.

Preferably, the pressure within said inflatable gasket is monitored while said inflatable gasket is in the inflated configuration.

Preferably, said method comprises generating an alert signal.

Preferably, said alert signal is generated in the event that said pressure falls outside a predefined range.

### Brief description of the drawings

Further features and advantages will become more apparent in the light of the following detailed description of a preferred, but non-limiting, embodiment of the invention. Such description is provided herein with reference to the annexed drawings, which are also supplied by way of non-limiting example, wherein:
- Figures 1a-1b show, in a very schematic manner, some possible general structures of the machine according to the present invention;
- Figure 2 shows a part of a machine in accordance with the present invention;
- Figure 3 shows a portion of Figure 2, sectioned along a substantially horizontal plane;
- Figure 4 shows a magnified detail of Figure 3;
- Figure 5 shows some elements of Figure 4, sectioned along a vertical plane;
- Figure 6 shows some elements of Figure 4 in a different operating configuration;
- Figure 7 shows some elements of Figure 6, sectioned along a vertical plane;
- Figure 8 is a sectional view along a horizontal plane of some elements of Figures 4-7;
- Figure 9 is a perspective view of some parts of the machine in the configuration shown in Figures 4-5;
- Figure 10 shows the same parts as shown in Figure 9 in the configuration of Figures 6-7;
- Figure 11 shows a block diagram of a pneumatic circuit included in the machine according to the present invention;
- Figure 12 shows a variant embodiment of the diagram of Figure 11;
- Figure 13 shows a variant embodiment of the diagram of Figure 12;
- Figure 14 shows a diagram that generalizes the embodiments shown in Figures 11-13.

### Detailed description of some preferred embodiments

With reference to the accompanying drawings, numeral 1 designates as a whole a machine in accordance with the present invention.

The machine 1 is a machine used for clinical/preclinical research, chemical or pharmaceutical applications.

For example, the machine 1 may be a machine intended for treating laboratory breeding cages used in the field of clinical/preclinical research.

In particular, the machine 1 may be one of: a washing machine; a disinfecting/sterilizing/decontaminating machine; a containment machine; an isolating machine.

The machine 1 may also perform two or more of the above-mentioned functions.

In general, all of the above-listed machine types share the need to ensure a hermetic sealing of the door by means of an inflatable tubular gasket.

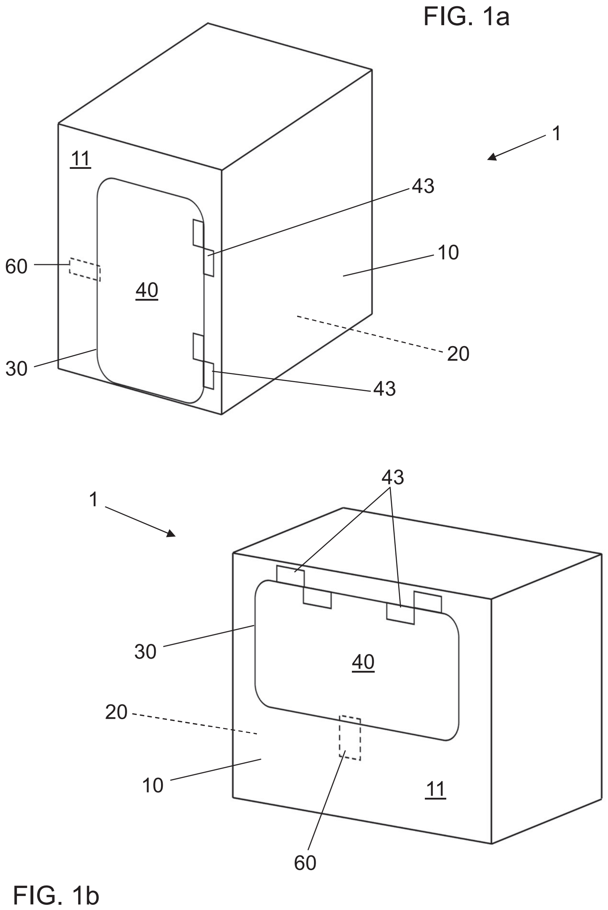

The machine 1 (Figures 1a-1b) comprises a box-shaped body 10, which internally defines a treatment chamber 20.

For example, the box-shaped body 10 may have a generic parallelepiped shape.

In the treatment chamber 20, one or more operations among those briefly mentioned above are carried out.

The treatment chamber 20 has at least one entrance 30 consisting of, in practical terms, an opening providing access to the treatment chamber 20 (i.e. an entrance leading into the box-shaped body 10).

For example, the entrance 30 may consist of an opening in a front or rear wall 11 of the box-shaped body 10.

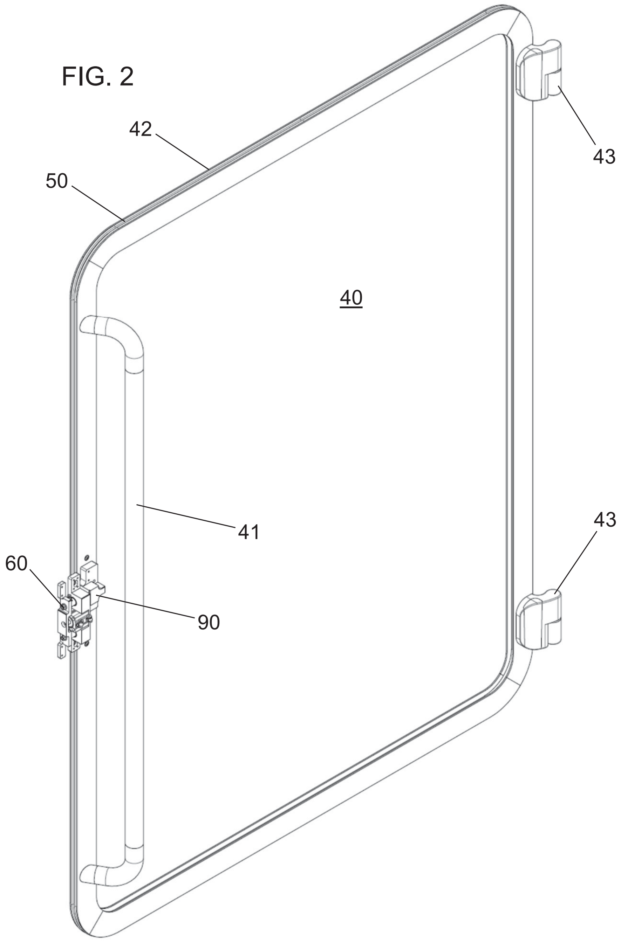

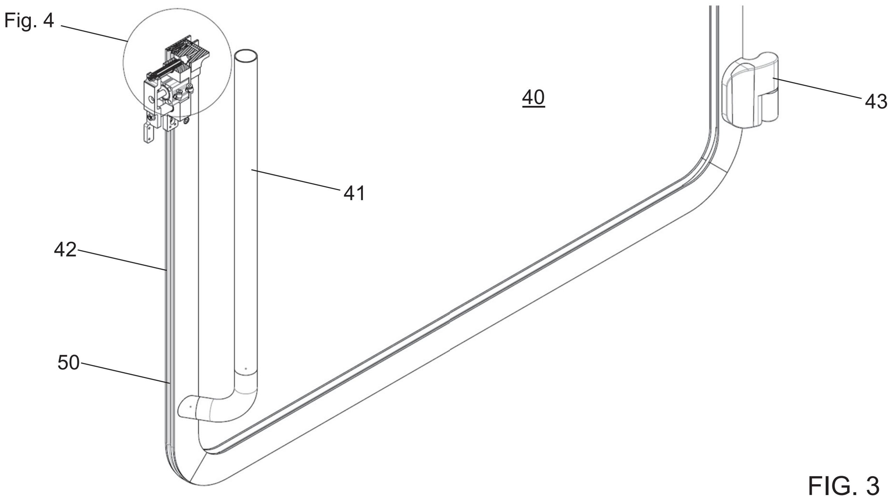

The machine 1 comprises a door 40 (Figures 1a-1b, 2-3), associated with the entrance 30.

The door 40 is controllable between a closed condition, in which it closes the entrance 30, and an open condition, in which it does not close the entrance 30.

For example, the door 40 may be hinged to the same wall in which the entrance 30 is formed (e.g. the front or rear wall 11), and can thus be opened/closed by pivoting about the hinges.

In one embodiment, the door 40 may be equipped with a bar handle 41 that can be easily gripped by a user, thus facilitating the opening/closing operations.

The machine 1 further comprises a tubular inflatable gasket 50, which extends along the perimeter of the door 40, in particular along the perimetric profile of the door 40.

For example, the inflatable gasket 50 is housed in a perimetric groove 42 of the door 40.

Preferably, the inflatable gasket 50 is of the "closed welded" type, i.e. it forms a continuous ring along its whole perimeter, allowing air to flow into it on a side interfaced with a duct supplying fluid under pressure coming from the at least one latch 60 (which will be described later on).

The inflatable gasket 50 is controllable between an inflated configuration and a deflated configuration.

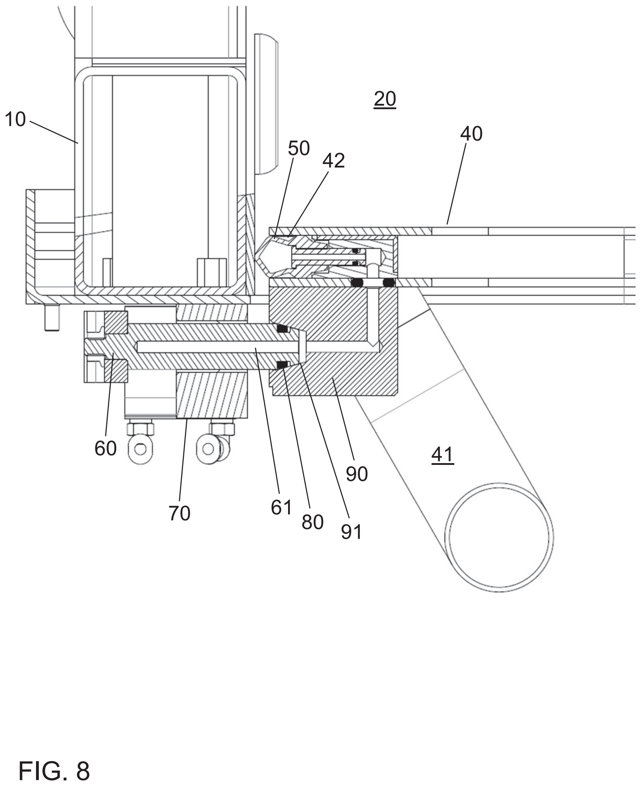

In the inflated configuration (Figure 8), the inflatable gasket 50 generates a sealed closure when the door 40 is in the closed condition. In particular, in this situation, the inflatable gasket 50 adheres to the surface of the inner profile of the entrance 30, thus sealingly separating the treatment chamber 20 from the environment outside the box-shaped body 10.

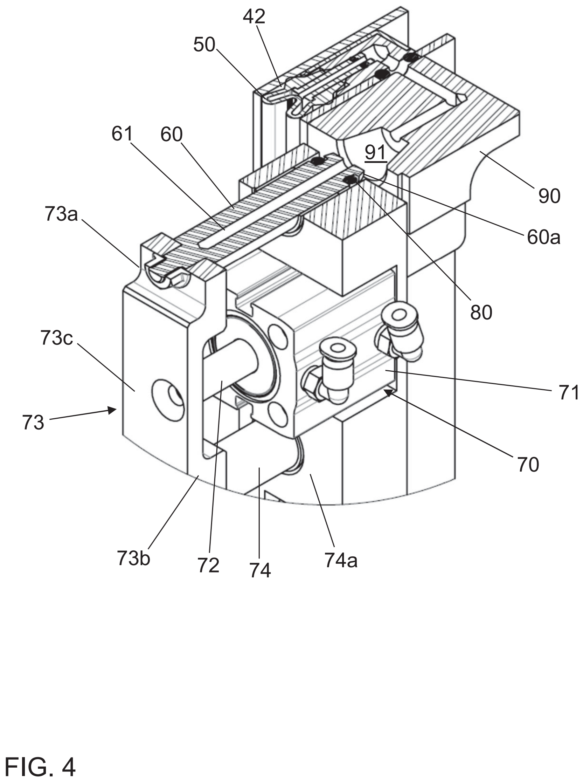

In the deflated configuration (Figure 4), the inflatable gasket 50 does not adhere to the entrance and does not provide such sealed separation.

The machine 1 further comprises at least one latch 60.

The at least one latch 60 is controllable between an operating condition and a rest condition.

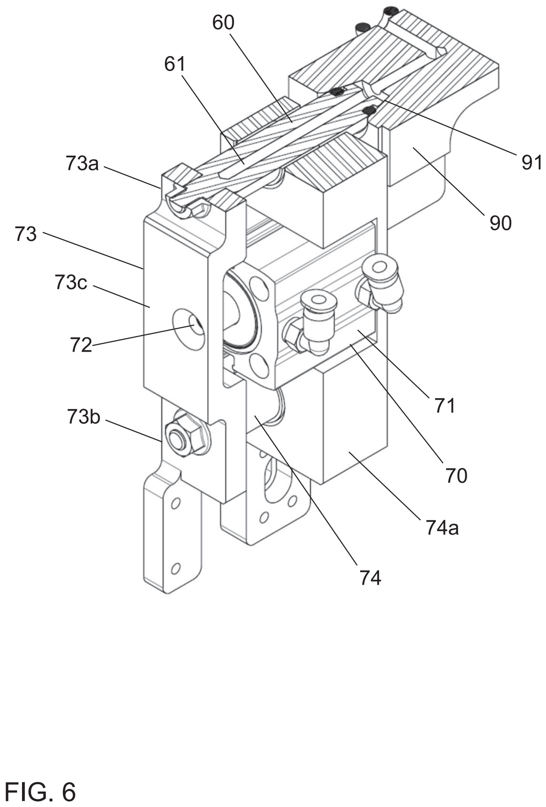

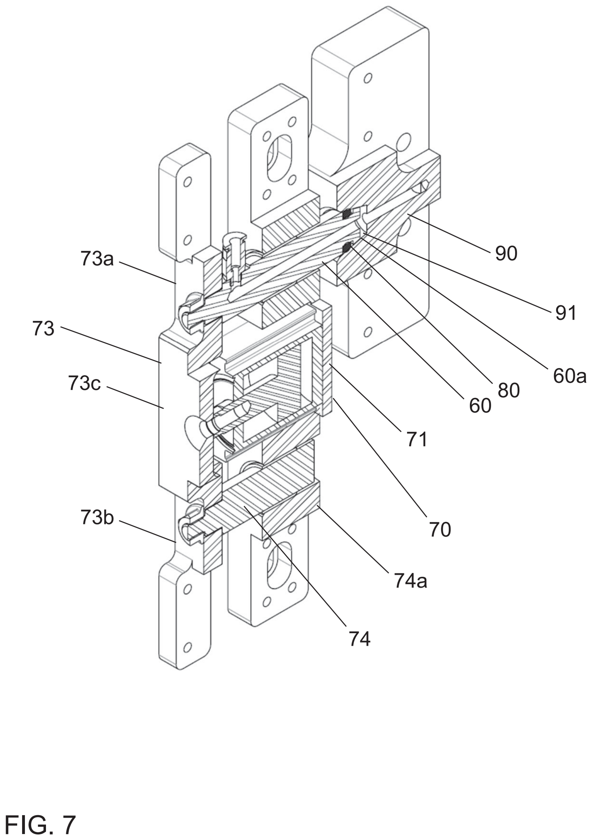

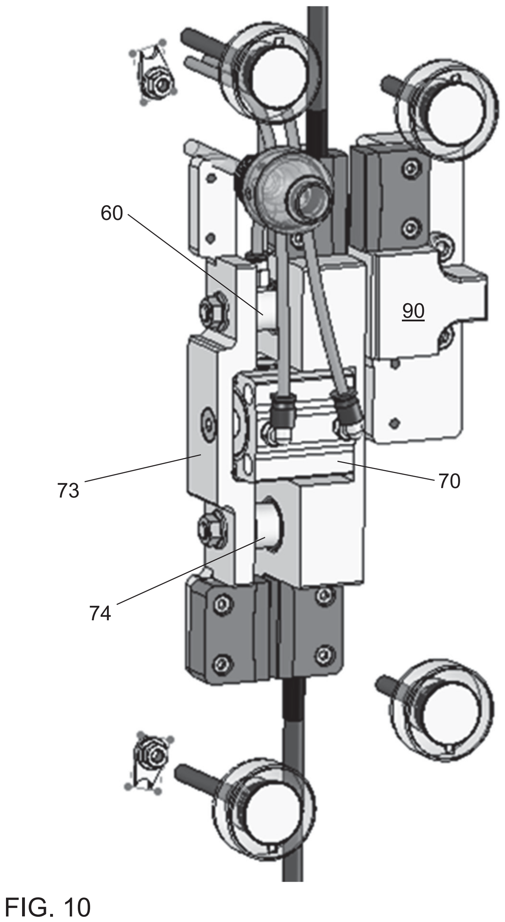

In the operating condition (Figures 6-8, 10), the at least one latch 60 locks the door 40 in the closed condition. In this situation, therefore, even without considering any resistance that may be opposed by the inflatable gasket 50, the door 40 cannot be opened by simply pulling/pushing it.

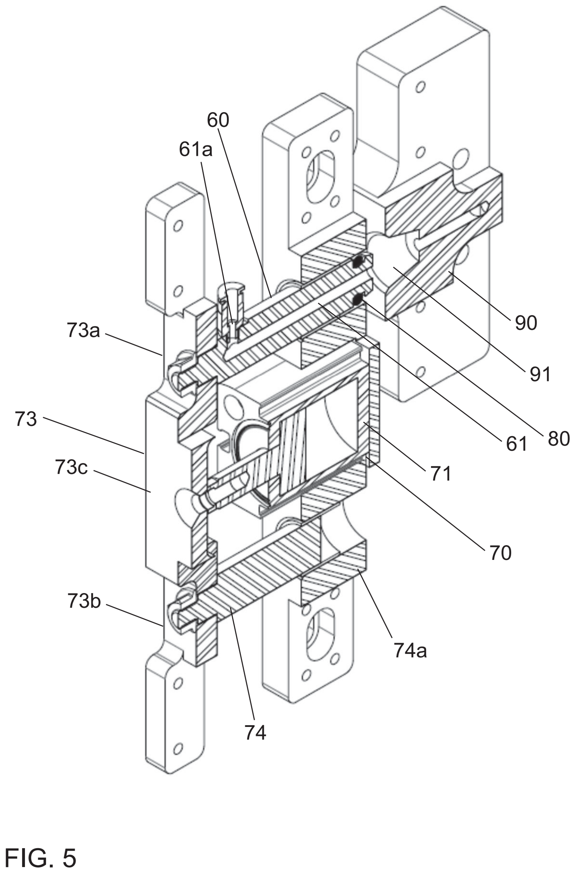

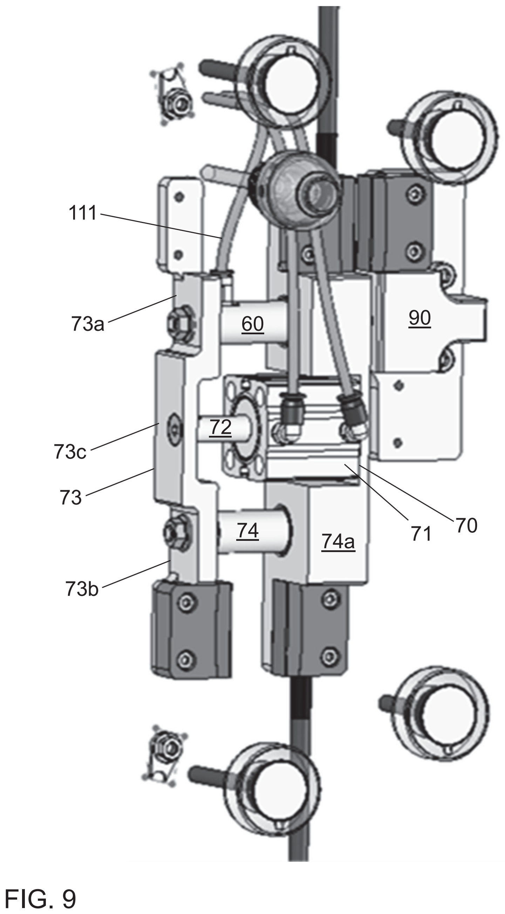

In the rest condition (Figures 4-5, 7, 9), the at least one latch 60 does not exert such locking action. In this situation, therefore, without considering any resistance that may be opposed by the inflatable gasket 50, the door 40 can be opened by simply pulling/pushing it.

For example, the at least one latch 60 is mounted to the box-shaped body 10 (e.g. to the front or rear wall 11), and, in the operating condition, intercepts a portion of the door 40 to exert said locking action. Such portion of the door 40 will be hereafter referred to as "striker block 90".

Figure 1a schematically shows a machine 1 with a treatment chamber 20 flush with a floor; the hinges 43 are sketched on the right, and the at least one latch 60 is drawn with a dashed line on the left, i.e. within the frame that surrounds the door 40 and on the left side.

Figure 1b schematically shows a machine 1 with a raised treatment chamber 20, a door 40 hinged at the top, and the at least one latch 60 at the bottom, drawn with a dashed line.

The machine 1 further comprises a pneumatic circuit 100.

Note that, in this context and in the appended claims, a pneumatic circuit is meant to be a circuit for fluid under pressure, in which a fluid, e.g. air or another suitable fluid, can circulate.

The pneumatic circuit 100 acts upon the inflatable gasket 50 to bring it into the inflated configuration.

The pneumatic circuit 100 preferably comprises a source 110 of fluid under pressure (e.g. air). Through suitable ducts, which will be further described below, said fluid under pressure is supplied to the inlet of the gasket 50 when the latter has to be brought into the inflated configuration.

In accordance with the invention, the at least one latch 60 houses part of the pneumatic circuit 100.

More particularly, the at least one latch 60, when it is in the operating condition, allows for fluidic communication between the source 110 and the inflatable gasket 50 in order to bring the latter into the inflated configuration.

On the contrary, when it is in the rest condition, the at least one latch 60 does not allow for fluidic communication between the source 110 and the inflatable gasket 50. In particular, when the at least one latch 60 is in the rest condition, the inflatable gasket 50 is deflated (i.e. it switches into the deflated configuration), tending to reach a point of pressure equilibrium with the outside environment.

As schematically shown in Figures 4-8, 11, the at least one latch 60 has an inner cavity 61.

The inner cavity 61 develops along a longitudinal tract of the at least one latch 60, preferably along a central axis of the at least one latch 60.

The inner cavity 61 has a first aperture 61a and a second aperture 61b.

The first aperture 61a is connected to the source 110 of fluid under pressure, e.g. through a duct 111.

The second aperture 61b is in fluidic communication with the inflatable gasket 50 when the at least one latch 60 is in the operating condition.

The inner cavity 61 of the at least one latch 60 is, therefore, a part of the pneumatic circuit 100.

Conversely, when the at least one latch 60 is in the rest condition, the second aperture 61b constitutes a free end of the at least one latch 60.

Preferably, the at least one latch 60 has a substantially cylindrical shape, with a tapered longitudinal end 60a that facilitates the movement into the operating condition.

The second aperture 61b is advantageously located at the tapered longitudinal end 60a.

In one embodiment, the machine 1 comprises a striker block 90.

Preferably, the striker block 90 is mounted to the door 40.

In particular, when the door 40 is in the closed condition, the striker block 90 faces towards the at least one latch 60.

The striker block 90 is in fluidic communication with the inflatable gasket 50.

When the at least one latch 60 is in the operating condition, the at least one latch 60 is in abutment with the striker block 90; in this situation, the striker block 90 allows for fluidic communication between the at least one latch 60 and the inflatable gasket 50.

In more detail, when the at least one latch 60 is in the operating condition, the flow of fluid under pressure coming from the source 110 enters through the first aperture 61a of the inner cavity 61, crosses the inner cavity 61, and exits through the second aperture 61b. Since the at least one latch 60 is in contact and in fluidic communication with the striker block 90, the flow of fluid under pressure exiting through the second aperture 61b crosses the striker block 90 and arrives at the inlet of the inflatable gasket 50. It is thus possible to switch the inflatable gasket 50 from the deflated configuration to the inflated configuration.

Furthermore, the coupling between the at least one latch 60 and the striker block 90 prevents opening the door 40, since it provides said locking of the door 40.

When the at least one latch 60 is in the rest condition, it is spaced apart from, and not in abutment with, the striker block 90.

Furthermore, when the at least one latch 60 is in the rest condition, and hence it is not coupled to the striker block 90, it does not prevent opening the door 40.

Preferably, the striker block 90 is at least partly shaped to match the shape of the at least one latch 60. In particular, the striker block 90 may have a recess 91, the shape of which is complementary to the shape of the tapered longitudinal end 60a of the at least one latch 60.

In one embodiment, the machine 1 comprises an auxiliary gasket 80 associated with said at least one latch 60 to provide a seal between the pneumatic circuit 100 and the inflatable gasket 50 when the latch 60 is in the operating condition.

In particular, the auxiliary gasket 80 may be mounted either to the at least one latch 60 (in proximity to the second aperture 61b) or to the striker block 90 (in the area where the at least one latch 60 is in abutment when it is in the operating condition), so as to generate a seal between the at least one latch 60 and the striker block 90. In practical terms, the auxiliary gasket 80 is, when the at least one latch 60 is in the operating condition, interposed between the at least one latch 60 and the striker block 90 in the abutment area. Thus, no leaks of fluid under pressure can occur between the at least one latch 60 and the striker block 90.

In order to move the at least one latch 60 between the rest position and the operating position, the machine 1 may be equipped with an actuator 70.

Preferably, the actuator 70 is a linear actuator, and imparts a substantially linear motion to the at least one latch 60, in the direction defined by the longitudinal development of the latter.

Advantageously, the actuator 70 may be mounted in proximity to the at least one latch 60; for example, the actuator 70 may be mounted to the inner portion of the frame that surrounds the entrance 30.

The actuator 70 includes a drive unit 71 and a sliding element 72, driven by the drive unit 71. In practical terms, the drive unit 71 is integrally connected to a part of the machine 1 (e.g. the box-shaped body 10) and causes the sliding element 72 to move relative to that part of the machine 1.

The drive unit 71 has a first chamber 71a and a second chamber 71b. When fluid under pressure is delivered into the first chamber 71a, the sliding element 72 will move into the extracted position, whereas when fluid under pressure is delivered into the second chamber 71b, the sliding element 72 will move into the retracted position.

The sliding element 72 may have a substantially cylindrical shape.

For example, the actuator 70 may be a pneumatic actuator. In a different embodiment, the actuator 70 may be, for example, an electromechanical actuator or another type of actuator, so long as it can move the at least one latch 60 as required.

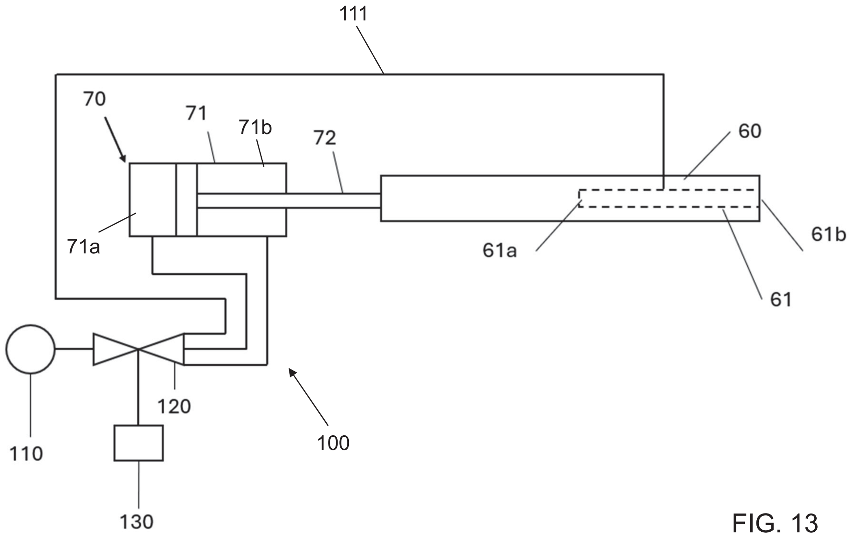

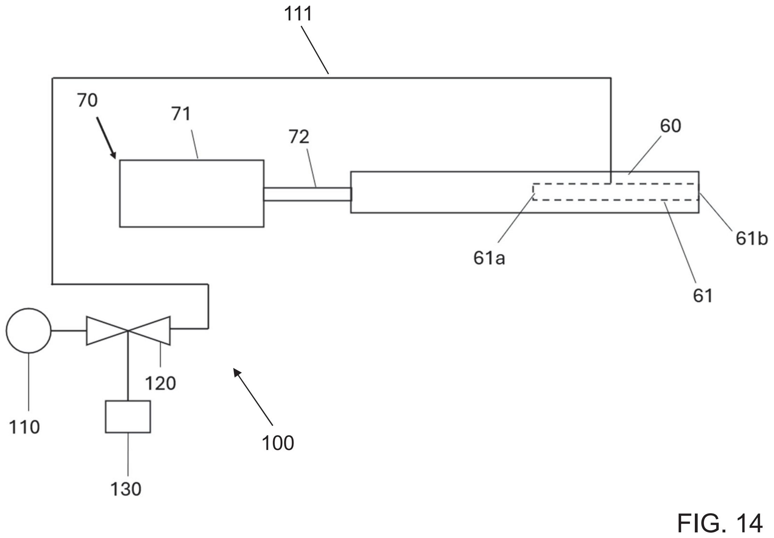

In one embodiment (Figures 13, 14), the at least one latch 60 coincides with the sliding element 72. Therefore, the extracted condition of the sliding element 72 will be the operating condition of the at least one latch 60, while the retracted condition of the sliding element 72 will be the rest condition of the at least one latch 60.

In complementary embodiments, the at least one latch 60 may be driven by the actuator 70 indirectly through kinematic means and/or linkages, while still preserving all of the above-mentioned features.

In one embodiment, for example, the at least one latch 60 does not coincide with the sliding element 72. Instead, it is connected to the sliding element 72, in particular through an elongate element 73 (Figures 4-7, 9-12).

The elongate element 73 may consist of, for example, a plate extending in a plane substantially orthogonal to the longitudinal development of the sliding element 72.

The elongate element 73 has a substantially central portion 73c, a first end 73a, and a second end 73b.

The second end 73b is opposite the first end 73a.

The substantially central portion 73c is interposed between the first and second ends 73a, 73b.

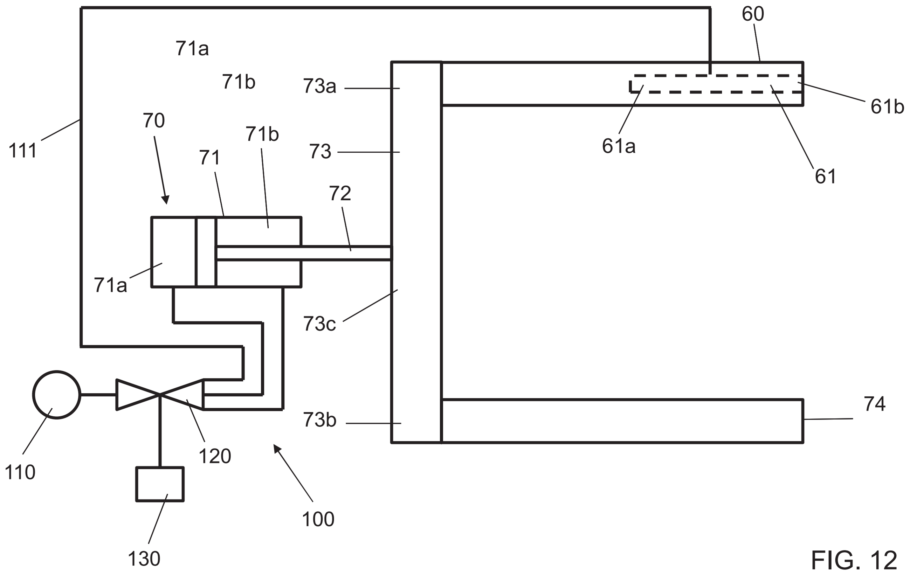

The sliding element 72 is engaged with the substantially central portion 73a of the elongate element 73; the at least one latch 60 is engaged with the first end 73a of the elongate element 73. Thus, the actuator 70 can impart the required motion to the at least one latch 60: with reference to, for example, Figures 4-7, 9-11, when the sliding element 72 is in the extracted position, the at least one latch 60 is in the rest condition; when the sliding element is in the retracted position, the at least one latch 60 is in the operating condition. Conversely, in the solutions shown in Figures 12-14 (which will be further described below), when the sliding element 72 is in the extracted position, the at least one latch 60 is in the operating condition; when the sliding element is in the retracted position, the at least one latch 60 is in the rest condition.

In order to be able to maintain the correct direction of motion over time, the machine 1 comprises an alignment element 74.

The alignment element 74 is coupled to the second end 73b of the elongate element 73. For example, the alignment element 74 may be provided in the form of a cylinder, integrally mounted to the second end 73b of the elongate element 73 and coupled to a seat 74a (integral with the drive unit 71), in which the alignment element 74 can slide. Due to this structure, the action exerted by the sliding element 72 can be distributed substantially evenly between the at least one latch 60 and the alignment element 74, thus maintaining, as aforesaid, the correct directions of motion.

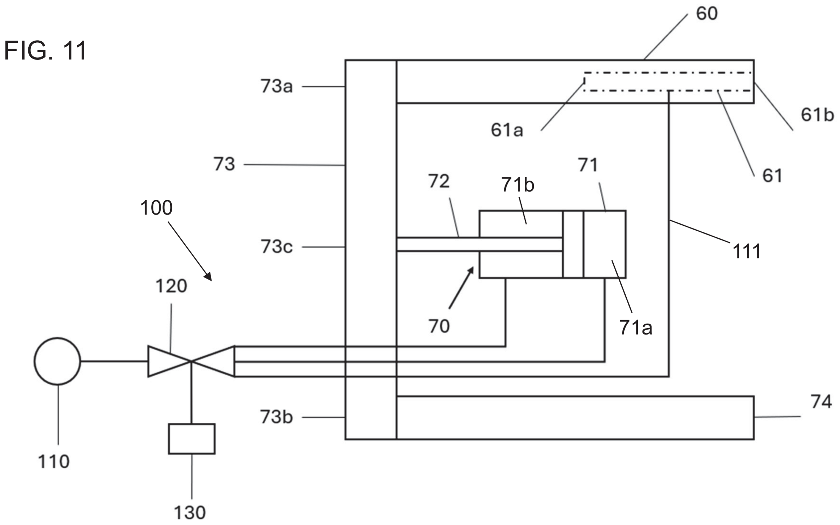

Figure 11 shows a diagram of the pneumatic circuit 100 included in the present invention.

The source 110 of fluid under pressure is connected, through a valve assembly 120, to the at least one latch 60 and to the actuator 70.

In general, the valve assembly is configured to at least selectively allow fluid under pressure to flow from the source 110 to the inflatable gasket 50.

More specifically, when the actuator 70 is a pneumatic actuator, the valve assembly 120 can be switched between at least three conditions.

In a first condition, the valve assembly 120 directs a flow of fluid under pressure towards the first chamber 71a of the drive unit 71 of the actuator 70, so as to bring the sliding element into the extracted position. According to the diagram of Figure 11, this results in the at least one latch 60 being brought into the rest condition.

In a second condition, the valve assembly 120 directs a flow of fluid under pressure towards the second chamber 71b of the drive unit of the actuator 70, so as to bring the sliding element into the retracted position. According to the diagram of Figure 11, this results in the at least one latch 60 being brought into the operating condition.

In a third condition, preferably subsequent to the second condition, the valve assembly 120 directs a flow of fluid under pressure towards the at least one latch 60, in particular into the cavity 61 of the latter, so as to bring the inflatable gasket 50 into the inflated configuration.

Advantageously, the valve assembly 120 is controlled by a control unit 130, which suitably controls the valve assembly 120 as a function of the operation that needs to be performed.

The valve assembly 120 may consist of, for example, a single valve interposed between the source 110, on one side, and the actuator 70 and the at least one latch 60, on the other side. In one variant embodiment, the valve assembly includes a plurality of valves, e.g. a first valve interposed between the source 110 and the actuator 70, and a second valve interposed between the source 110 and the at least one latch 60.

Figure 12 shows a construction diagram which is alternative to the one shown in Figure 11, wherein the assembly including the at least one latch 60, the elongate element 73 and the alignment element 74 is moved by the actuator 70, through the sliding element 72, so that:
when the sliding element 72 is in the extracted position (fluid under pressure delivered into the first chamber 71a), the at least one latch 60 is in the operating condition; in this situation, fluid under pressure is then delivered through the cavity 61 of the at least one latch 60, so as to inflate the inflatable gasket 50;
when the sliding element 72 is in the retracted position (fluid under pressure delivered into the second chamber 71b), the at least one latch 60 is in the rest condition.

As mentioned above, Figure 13 shows a further possible embodiment, wherein the at least one latch 60 essentially coincides with the sliding element 72, or anyway is directly mounted to the sliding element 72 without interposition of the elongate element 73. The principle of operation is the same as shown in Figure 12, the only difference being that, as aforesaid, the actuator 70 acts upon the at least one latch 60 directly, as opposed to via the elongate element 63.

Figure 14 shows a more general diagram, wherein the actuator 70 is not necessarily a pneumatic one; the pneumatic circuit 100 is used for inflating the inflatable gasket 50, and may not be used for moving the at least one latch 60. It should be noted that this generalization of the actuator type is also applicable to the embodiments shown in Figures 11-12, wherein the sliding element 72 does not act upon the at least one latch 60 directly, but via the elongate element 73 (regardless of whether the extracted position of the sliding element 72 corresponds to the operating condition or to the rest condition of the at least one latch 60).

As far as the operation of the machine 1 is concerned, the following should be noted.

In a normal initial situation, the door 40 is in the open condition, with the inflatable gasket 50 in the deflated configuration and the at least one latch 60 in the rest condition.

In order to set the machine 1 in operation, it is necessary to establish a sealed separation between the treatment chamber 20 and the outside environment.

For this purpose, the door 40 is brought into the closed condition, e.g. by turning it about the axis of its hinges.

When the door 40 is in the closed condition, the at least one latch 60 is brought into the operating condition: for example, the drive unit 71 acts upon the sliding element 72, guiding it towards the retracted position (Figure 11) and, through the elongate element 73, dragging the at least one latch 60 towards the operating condition. In the embodiments shown in Figures 12-14, the operating condition of the at least one latch 60 is obtained by moving the sliding element 72 into the extracted position.

The tapered longitudinal end 60a is now in contact with the striker block 90, and the tightness of the fluidic connection is ensured by the auxiliary gasket 80. At the same time, the door 40 is locked due to the coupling between the at least one latch 60 and the striker block 90.

At this point, the source 110 starts pumping fluid under pressure through the duct 111, the cavity 61 of the at least one latch 60, the striker block 90, up to the inflatable gasket 50, which will thus be switched from the deflated configuration to the inflated configuration.

This will generate the sealed closure of the door 40.

When the machine 1 no longer needs to operate, or anyway when the door 40 has to be opened, the following procedure is carried out.

The at least one latch 60 is brought into the rest condition; in this manner, the fluid under pressure in the inflatable gasket 50 will come out, bringing the inflatable gasket 50 into the deflated configuration. Furthermore, since the at least one latch 60 is in the rest condition, there is no longer any interference with the striker block 90, and the door 40 is no longer locked. In the embodiment of Figure 11, the rest condition of the at least one latch 60 is obtained by bringing the sliding element 72 into the extracted position; in the embodiments of Figures 12-14, the rest condition of the at least one latch 60 is obtained by bringing the sliding element 72 into the retracted position.

When the inflatable gasket 50 has been sufficiently deflated, the door 40 can be opened in order to, for example, gain access to the treatment chamber 20.

Advantageously, it is envisaged that, while the inflatable gasket 50 is in the inflated configuration, the pressure within the inflatable gasket 50 is monitored - for example, by a suitable pressure sensor. Should the pressure fall outside a predefined range, an alert signal will be generated. The latter may be, for example, an audible and/or visual signal for an operator, or an informative signal causing, through the control unit 130, the execution of dedicated actions. Depending on the current condition of the machine, different actions may be performed. For example, a message may be shown on the external display of the machine, or the operating cycle of the machine may be aborted, or a signal may be generated indicating that the isolation ("barrier") between the inside of the machine and the outside environment has failed.

The invention attains some important advantages.

First of all, it considerably simplifies the structure of the hinge(s), which can be standard commercially available hinges, whereas in prior-art solutions said structure was required to allow the supply of air under pressure for the inflatable gasket.

Moreover, just one signal is necessary to detect when the door is closed and in position and when the gasket is inflated. In particular, this is due to the fact that the inflating function will only be turned on if the latch is closed and in the correct position, and therefore the detection of an inflated gasket will necessarily imply that the latch is closed and in the correct position. This means, in turn, that the door is closed, locked and sealed. As a matter of fact, the gasket inflation function may fail because of a punctured gasket, a non-transmitted pressurized flow, a non-operated latch, or a latch operated with an improperly closed door.

In addition to the above, in case of emergency the door can be easily opened by discharging the pressure to immediately deflate the gasket and retract the cylinder that drives the latch - this being possible also from the inside and in the absence of compressed air and electric energy.

A further advantage lies in the fact that, due to the solution proposed in the present invention, it becomes essentially impossible to unintentionally inflate the gasket, thus damaging it, when the door is open, since the gasket can only be pressurized when the door is in position, i.e. in abutment with, and internal to, the frame.

Yet another advantage lies in the fact that pressurization and depressurization of the tubular gasket are considerably faster than in the solution where the fluid flow passes through the hinge, because in this case a direct circuit is used, with no bottlenecks or labyrinths.

In general, the Applicant observes that all of the above advantages are achieved through a solution which is less complex than prior-art ones, resulting in lower costs, better reliability and safety, and, on the whole, higher effectiveness and efficiency.

## Claims

1. Machine for washing and/or decontaminating objects, for applications in the chemical, pharmaceutical and/or clinical/preclinical research fields, comprising:
a box-shaped body (10), defining a treatment chamber (20), said treatment chamber (20) having at least one entrance (30);
a door (40), associated with said entrance (30) and controllable between a closed condition, in which it closes said entrance (30), and an open condition, in which it does not close said entrance (30);
an inflatable gasket (50), extending along a perimeter of said door (40), said inflatable gasket (50) being controllable between an inflated configuration, in which it generates a sealed closure when said door (40) is in the closed condition, and a deflated configuration;
at least one latch (60), controllable between an operating condition, in which it locks the door (40) in the closed condition, and a rest condition, in which it exerts no locking action;
a pneumatic circuit (100), acting upon said inflatable gasket (50) to bring it into the inflated configuration;
wherein said at least one latch (60) houses part of said pneumatic circuit (100).

2. The machine according to claim 1, wherein said at least one latch (60), when it is in the operating condition, allows for fluidic communication between a source (110) of fluid under pressure and said inflatable gasket (50) in order to bring the latter into the inflated configuration, wherein said at least one latch (60), when it is in the rest condition, preferably does not allow for fluidic communication between the source (110) of fluid under pressure and the inflatable gasket (50).

3. The machine according to claim 2, wherein said at least one latch (60) has an inner cavity (61) having a first aperture (61a) and a second aperture (61b), wherein said first aperture (61a) is connected to said source (110) of fluid under pressure, and said second aperture (61b) is in fluidic communication with said inflatable gasket (50) when said at least one latch (60) is in the operating condition.

4. The machine according to any one of claims 2-3, wherein said pneumatic circuit (100) comprises a valve assembly (120), configured to at least selectively allow fluid under pressure to flow from the source (110) to the inflatable gasket (50).

5. The machine according to any one of the preceding claims, further comprising an actuator (70) configured to move said at least one latch (60) between the operating condition and the rest condition, wherein said actuator (70) is preferably a linear actuator equipped with a drive unit (71) and a sliding element (72) driven by said drive unit (71),
wherein said at least one latch (60) is connected to said sliding element (72).

6. The machine of claim 5, wherein said at least one latch (60) is connected to said sliding element (72) through an elongate element (73), wherein said elongate element (73) has a substantially central portion (73c), a first end (73a), and a second end (73b) opposite said first end (73a), wherein:
the sliding element (72) is engaged with the substantially central portion (73a) of the elongate element (73);
the at least one latch (60) is engaged with the first end (73a) of the elongate element (73);
the machine (1) comprises an alignment element (74) coupled to the second end (73b) of the elongate element (73).

7. The machine according to any one of claims 4-6, wherein said actuator (70) is a pneumatic actuator, wherein said valve assembly (120) is preferably configured to be controlled between:
a first condition, in which it directs a flow of fluid towards said actuator (70) to bring said at least one latch (60) into the rest condition;
a second condition, in which it directs a flow of fluid towards said actuator (70) to bring said at least one latch (60) into the operating condition;
a third condition, in which it directs a flow of fluid towards said at least one latch (60) to bring said inflatable gasket (50) into the inflated configuration.

8. The machine according to any one of the preceding claims, wherein:
said at least one latch (60) is mounted to said box-shaped body and, in said operating condition, intercepts a portion of said door so as to exert said locking action
and/or
said at least one latch (60) has a substantially cylindrical shape, with a tapered longitudinal end (60a) that facilitates the movement into the operating condition.

9. The machine according to any one of the preceding claims, wherein:
said machine further comprises an auxiliary gasket (80) associated with said at least one latch (60) to provide a seal between the pneumatic circuit (100) and the inflatable gasket (50) when the latch (60) is in the operating condition
and/or
said inflatable gasket (50) is housed in the perimeter of the door (40), preferably in a perimetric groove (42) of said door (40).

10. The machine according to any one of the preceding claims, comprising a striker block (90), wherein said striker block (90) is in fluidic communication with said inflatable gasket (50), wherein, when the at least one latch (60) is in the operating condition, said at least one latch (60) is in abutment with said striker block (90) and said striker block (90) allows for fluidic communication between said at least one latch (60) and the inflatable gasket (50), wherein said striker block (90) is preferably at least partly shaped to match the shape of said at least one latch (60).

11. The machine according to claim 10 when dependent on claim 8, wherein said auxiliary gasket (80) is mounted to either said at least one latch (60) or said striker block (90) to provide a seal between said at least one latch (60) and said striker block (90).

12. The machine according to any one of claims 10-11, wherein said at least one latch (60), when it is in the rest condition, is spaced apart from, and not in abutment with, said striker block (90).

13. Method of operation of the machine according to any one of the preceding claims, said method comprising:
bringing said door (40) into said closed condition;
bringing said at least one latch (60) into the operating condition, so as to lock said door (40) in the closed condition;
activating said pneumatic circuit (100) to bring said inflatable gasket (50) into the inflated configuration through said at least one latch (60) and generate a sealed closure of said door (40).

14. The method according to claim 13, further comprising a step of opening said door (40), said step of opening comprising:
moving said at least one latch (60) from the operating condition to the rest condition;
bringing said inflatable gasket (50) into the deflated configuration;
moving said door (40) from the closed condition to the open condition.

15. The method according to claim 13 or 14, further comprising:
while said inflatable gasket (50) is in the inflated configuration, monitoring a pressure within said inflatable gasket (50);
generating an alert signal in the event that said pressure falls outside a predefined range.
